# EUROPEAN PATENT APPLICATION

(11) **EP 2 960 654 A1**
(43) Date of publication of application: **30.12.2015**
(21) Application number: 14230006.0
(22) Date of filing: 27.06.2014
(51) Int. Cl.: G01N 33/68, G01N 33/88

(54) **A method for detecting EP3 antibodies**

(71) Applicant: Ludwig-Maximilians-Universität München, 80539 München (DE)
(72) Inventor: von Schönfeldt, Viktoria, 82131 Gauting (DE); Jeschke, Udo, 80992 München (DE); Rogenhofer, Nina, 80639 München (DE); Thaler, Christian, 82152 Planegg (DE)
(74) Representative: Hoefer & Partner Patentanwälte mbB

(57) **Abstract**

A method for the detection of EP3-autoantibodies comprises contacting a sample with EP3 protein or an immunologically active fragment thereof and assessing for formation of an immune complex between the autoantibody and EP3.

## Description

The present invention is concerned with a method for assessing anti-EP3 activity or EP3 receptor autoantibodies in a patient, in particular a patient having a risk for miscarriage or premature birth.

Miscarriage is a common complication of pregnancy, it is the spontaneous loss of a fetus before it has reached viability. Miscarriage occurs in about 15 to 20% of recognized pregnancies. The probability of a further miscarriage increases with the number of miscarriages. Underlying causes are often not found for recurrent miscarriages. There are some risk factors like increased maternal age or genetic abnormalities; immunological reasons, thrombophilia, infections, anatomical conditions can be further reasons.

Miscarriage is very cumbersome for the concerned parents and it is highly desirable to find causes for miscarriage and possible treatment regimes. Investigations have shown that antiphospholipid antibodies could be associated with early miscarriages and maternal mortality and it has been proposed to screen women for inherited thrombophilias including factor V Leiden, factor II (prothrombin) gene mutation and protein S.

Reasons for habitual abortion can be chromosomal causes, for example translocations, hormonal or metabolic abnormalities, like hyperprolactinaemia, thyroid dysfunction, luteal phase defect, hyperanthrogenaemia, PCO syndrome. Further reasons can be autoimmune diseases like lupus erythematosus, or infections with microorganisms like urea plasms, chlamydia and toxoplasm.

F. Nawroth et al. (Frauenarzt 47 (2006), no. 4, pages 300-305) describe that in a number of cases of habitual abortions antiphospholipid antibody syndrome can be a reason and can be treated with acetylsalicylic acid. Clinical tests are proposed for anticardiolipin antibodies, lupus anticoagulants, and β₂-glycoprotein-I-antibodies.

L. Sabatini et al. (Clinical Chemistry 53:2 228-232, 2007) found that increased plasma concentrations of antiprothrombin antibodies are related to antiphospholipid syndrome and might also cause miscarriages.

However, there is still a high number of habitual abortions or recurrent miscarriages that have no clearly associated underlying cause.

Therefore, it was an object of the present invention to provide a method for assessing the risk for miscarriage, in particular recurrent miscarriage and by providing a diagnostic tool allowing treatment or prevention.

The inventors surprisingly found that in patients with recurrent miscarriages anti-EP3 activity was significantly higher than in controls and that there is a connection between the presence of anti-EP3 antibodies in serum of patients and recurrent miscarriages. In a study it was determined that 15 to 30% of patients with RSA have EP3 autoantibodies.

Thus, the present invention provides a method for assessing the risk for miscarriages and habitual abortions by determining if anti-EP3 antibodies are present in the serum of a patient. In case anti-EP3 antibodies are found, prevention of abortion and treatment of such women becomes possible, for example by dosing acetylsalicylic acid and/or heparin.

An assumption is that EP3 antibodies have an influence in the thrombotic system, in particular in the inhibition of the digestion of fibrin clots in the spiral arteria of the decidua which is counteracted by antithrombotica.

Without being bound by theory, an explanation for the link between EP3 autoantbodies and abortions could be that EP3 inhibits cAMP synthesis and has influence to the synthesis of progesteron and hCG and that the binding of EP3 autoantibodies to the EP3 receptor is a cause for abortion because of inhibition of hCG and progesteron synthesis.

EP3 is one of the four G protein-coupled E prostanoid receptors which can bind prostaglandin E2, a member of the autacoid family of lipid mediators. Prostaglandin E2 plays a major role in the arachidonic acid metabolism, it has various functions and is expressed in renal vessels, uterine, collecting duct. From US 2011/0130347 A1 it was known that the activity of EP3 can be modulated to treat reproduction disorders.

As EP3 is highly expressed in tissues of the male and female reproduction system, in tissues which are involved in inflammatory processes, in cardiac tissue, in the thrombotic system and other tissues, anti-EP3 autoantibodies might be involved in other pathogenic conditions and, therefore, it is another object of the present invention, to provide an assay for detecting the presence of EP3 antibodies in samples and tissues of subjects, in particular humans as a diagnostic tool.

As EP3 has a function also outside the reproduction system, for example it is involved in the inflammatory process and with clot and plaque formation and dissolution, the determination of EP3 autoantibodies may also play a role in the diagnostic of other diseases. Therefore, according to the present invention also a method for assessing the presence of anti-EP3 activity and/or EP3 autoantibodies is provided.

### Definitions

EP3 is a receptor for human prostaglandin E2. In the present application the term "EP3" or "EP3 protein" is used for the full length protein as well as fragments having immunological property. The sequence is known and can be found in gene databases, such as GenBank.

The term "immunologically active fragment" of EP3, when used in the present application refers to fragments of EP3 that have immunological activity, i.e. are bound by EP3 antibodies, in particular EP3 autoantibodies. Fragments of EP3 are in particular those proteins or peptides comprising at least one epitope of EP3, for example sequences having at least 6, in particular at least 8, preferable at least 15 or more, preferred at least 25 contiguous amino acids that form an epitope. The epitope can be flanked on either or both sides by further amino acids of EP3 or neutral or enhancer sequences. Methods to identify and prepare immunologically active fragments of EP3 are known to the skilled artisan, for example by providing a protein or fragment having a sequence of at least 6, preferably 12 to 50 or more amino acids of human EP3 and by testing the fragment for its binding properties. A test for identifying immunologically active fragments can comprise contacting a fragment with an EP3 antibody and analyzing if binding occurs at least with the same selectivity and strength as with natural EP3.

Any EP3 that is available and has immunological properties as natural EP3 can be used for the present invention, it can be obtained from natural sources but normally is recombinant EP3 or an immunologically active fragment thereof.

Immune complex between an autoantibody and EP3 refers to any complex between autoantibodies to be detected and an epitope of EP3, which can be present on a full length protein or an immunologically active fragment thereof, such as a peptide comprising at least one EP3 epitope.

The terms "miscarriage" and "abortion" are used interchangeably in the present application. Miscarriage or abortion means the premature end of a pregnancy with fetal loss which occurs within the 20 to 24 first weeks of pregnancy. Recurrent spontaneous abortion (RSA) is usually defined as the loss of three or more consecutive pregnancies at less than 20 to 24 weeks of pregnancy.

"EP3 autoantibody" is an antibody produced by the immune system of a subject, preferably a mammal, in particular a human being which binds to EP3. Autoantibodies are endogenously built but are damaging for the body.

"Immunoassay" is any test method using immunological binding for the detection of a substance.

An "immunometric method" is any format of a method for detection of a substance by using immunological binding or reactivity, in particular by capturing an antibody, contacting it with a measurable label and detecting the presence and/or amount of bound label.

A "ligand" is a substance that binds to another component in a specific way, like biotin to avidin, an antigen to an antibody etc.

The term "sample" in the present application is used for any type of sample obtained from a subject such as a mammal, in particular a human being, and it can be any type of sample as known in the diagnostic field and can be any type of substance or composition that is used in a test for the determination of presence of EP3 autoantibodies, such as cells, tissues or a solid, fluid, liquid, obtained from a subject. Typically a sample as used in the present invention is any type of solution, solid or tissue to be tested, in particular a body liquid such as blood, lymph, serum, urine, liquor, cells, tissue. Particularly serum, plasma, blood, urine, renal or uterine cells are used for the detection of EP3 autoantibodies.

The terms "determination" or "detection" of the presence of EP3 autoantibodies when used in the present application include quantitative and/or qualitative methods.

The term "marker" or "label" refers to any substance that is detectable and/or measurable by physical, chemical, biochemical, or immunological means. It includes substances that are suitable for qualitative and/or quantitative detection.

### Figure

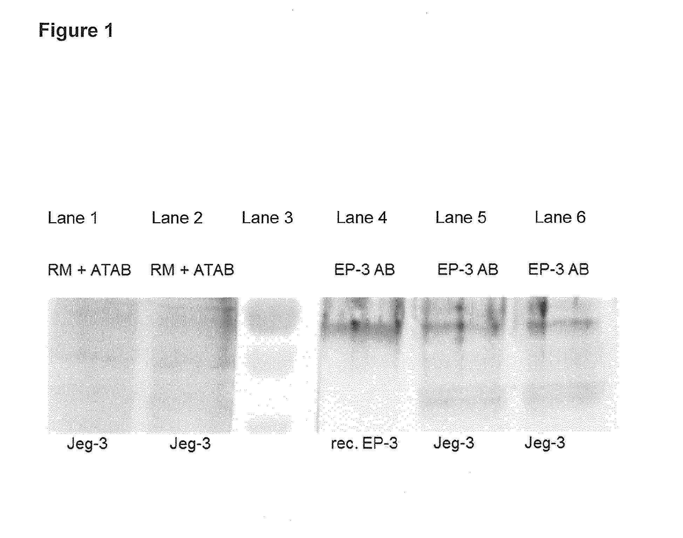

Figure 1 shows the results for the identification of anti-EP3 antibodies (ATAB) by Western Blot.

In a first aspect the present invention is concerned with a method for the determination of the presence of EP3 autoantibodies in a sample, wherein the method comprises contacting the sample with an EP3 protein or fragment thereof and determining if an immune complex between an autoantibody against EP3 and the EP3 protein or EP3 fragment has formed.

The determination of the presence of EP3 autoantibodies can be done with any known method for determining or analyzing the formation of complexes between a ligand and an antibody. Many formats for such tests are available und suitable methods are well-known to the skilled person. Generally suitable is any method that allows the visualization of a complex formation. Detection can be achieved by either labelling one of the partners of the complex, such as the EP3 protein or fragment, or, by labelling another ligand, that specifically binds to one of the partners of the complex.

Labels or markers that are easily detectable are well-known and include for example radioactive labels, such as radioactive isotopes of sulfur, phosphor, hydrogen, carbon, nitrogen etc.; fluorescent groups, such as fluorescein, or fluorescent proteins; chemoluminescent groups; metal colloids such as gold or silver; and enzymes such as horse radish peroxidase, alkaline phosphatase, β-galactosidase or other suitable enzymes.

The binding partner - EP3 protein or a fragment thereof - can be in solution or in immobilized form, i.e. bound to a solid phase which can be a carrier, support or matrix such as the surface of a microplate or a microparticle or nanoparticle.

The immobilization is done as is known to the skilled person, usually via a binding group. Many methods for immobilization are known and available.

For the method of the present invention, EP3 protein can be used as full-length protein. The protein is commercially available. Furthermore, it is possible to use an immunologically active fragment of EP3, i.e. a fragment, that binds with EP3 autoantibodies in a strength and a specificity that is comparable to the binding of EP3 protein with autoantibodies that can be obtained from samples. Fragments can be sequences that comprise at least one epitope of EP3.

Many formats of diagnostic methods for the determination of an antibody or an autoantigenic activity are available. Suitable methods are for example blotting methods such as dot blot or western blot, flow cytometry, or immunometric methods like sandwich methods, such as enyme linked immunosorbent assay (ELISA) etc.

One embodiment of the diagnostic method of the present invention comprises a dot blot method. In a dot blot method generally EP3 protein of full length or an immunologically active fragment thereof is dotted on a carrier or support, such as a membrane, for example a nitrocellulose membrane. Then, the sample obtained from the subject is added and incubated for a period that allows antibodies contained in the sample to bind to the EP3 protein or fragment on the membrane. The binding is then detected by adding a detection agent for a qualitative and/or quantitative assessment. Washing steps are usually carried out before adding the detection agent, to avoid unspecific signals.

The detectable ligand can be any substance that binds specifically with the partner of the complex to be detected, i.e. the autoantibody and has a detectable part or provides a binding site for a detectable label. In a preferred embodiment, therefore, the detectable ligand is a labelled or labelable antibody that binds to the FC part of the autoantibody. In case of a human sample, preferably the ligand is antihuman IgG and/or IgM. Fragments of such antibodies can also be used as is known in the art, for example Fv-, F_{ab}- or F(ab')₂-fragments. The detectable part of the ligand or detectable label is a label that is detectable with well-known methods as outlined above.

In a preferred embodiment a label enhancing the signal, such as an enzyme, is used, which can be coupled to the detectable ligand by a linking unit, for example a biotin-streptavidin bridge.

When an enzyme is used, for detection, a substrate for the enzyme is added which is detectable after reaction with the enzyme. Suitable substrates for enzymes that are used in immunoassays are well-known in the art. In one preferred embodiment, alkaline phosphatase is used as enzyme and 5-bromo-4-chloro-3-indolylphosphate/nitroblue tetrazolium chloride (NBT) is used as substrate which produces a blue-purple precipitate when added. This substrate is particularly useful because it provides a very high sensitivity and has sustained light emission characteristics. In another embodiment horseradish peroxidase is used as enzyme, a suitable substrate in this case is 3,3',5,5'-tetramethylbenzidine (TMB).

The dot blot method can comprise further blocking and washing steps, as is known. Blocking agents and washing solutions and buffers are well-known in this field.

In one embodiment after having loaded the membrane with EP3 protein or fragments thereof, a blocking agent can be added to saturate reminding binding sites. Inhibitory agents for this use are very well-known in the art, casein being an example.

In a further embodiment anti EP3 activity or EP3 autoantibodies can also be detected using a Western blot method. Western blot methods are well-known in the art. This method is particularly useful for a qualitative assay. In a Western blot method, samples are loaded on a gel, EP3 or a fragment thereof is added and the formation of a complex is detected via a detectable label.

A further method for detecting anti EP3 activity in a sample is flow cytometry. A suspension comprising particles carrying EP3 protein or fragments thereof which have been reacted with a sample such that antibodies present in the sample could bind to EP3 and wherein the formed complexes are labeled with fluorescent dyes are subjected to a flow cytometric analysis. Various immunoflurescent dyes are known for this technology and can be used in known manner. The sample suspension is fed to a tube or flow cell and when entering the flow channel, the particles pass through a focused laser beam one particle at a time. Light scatter and fluorescence light are captured and converted to electrical signals.

Furthermore, any type of immunometric assay can be used to determine the presence and amount of EP3 autoantibodies in a sample. One example is an assay wherein capture units are immobilized EP3 proteins or fragments as defined above and wherein measurable labels are preferably conjugates of anti human IgG and/or IgM or fragments thereof and enzymes as defined above.

The detection of EP3 autoantibodies can be carried out in any type of sample. For detection of a risk for miscarriage preferably the immunoassays carried out with a sample of a non-pregnant woman. This allows to carefully monitor the course of pregnancy and treatment at the necessary time, if antibodies and, thus, a risk are detected.

The method for detection of EP3 antibodies is useful for assessing the risk for any EP3 related disease. Thus, the sample can be a sample from any human being where a risk for an EP3 antibody dependent disease shall be assessed. As EP3 is involved in male and female reproduction system in general, in inflammatory processes, in clot and plaque formation and dissolution, atheriosclerotic processes, antiphospholipid syndrome etc., the determination of EP3 autoantibodies may also play a role in the diagnostic of dysfunctions or diseases in this regard. The method of the present invention, therefore, provides a valuable tool for the diagnosis of diseases related to or involved with inflammatory processes, diseases or dysfunctions of the reproduction system, diseases and dysfunctions of the thrombotic system, diseases related to or involved with atherosclerotic processes and other EP3 related conditions and pathogenicities. Therefore, according to the present invention also a method for assessing the presence of anti-EP3 activity and/or EP3 autoantibodies is provided, wherein a signal is a hint to an EP3 related disease, dysfunction or condition.

In a further embodiment the present invention provides a kit for carrying out a detection assay for EP3 antibodies in a sample. The kit comprises at least one or more EP3 proteins or fragments thereof, a conjugate comprising an antibody binding unit, such as antihuman IgG and/or antihuman IgM, and a detectable unit, such as an enzyme, and optionally buffer and/or wash solutions. In case the detectable unit comprises an enzyme, substrate for the enzyme is also included.

Furthermore, a kit for assessing a risk for miscarriage or premature birth is provided which comprises one or more EP3 proteins or immunologically active fragments thereof, a conjugate comprising an antibody binding unit and a detectable unit and optionally buffer and/or wash solutions.

Preferably the antibody binding unit used in the kits defined above is antihuman IgG and/or antihuman IgM or a fragment thereof. The detectable unit is preferably an enzyme such as alkaline phosphatase or horse radish peroxidase. If an enzyme is used as detectable unit, the kit further comprises a substrate for the enzyme that creates a detectable signal by reaction with the enzyme.

The present invention provides a method and a kit for detecting anti EP3 activity and EP3 antibodies. The presence of EP3 antibodies is an indication for EP3 related diseases and in particular for a risk for miscarriage or RSA respectively. Having determined the risk, treatment is possible.

The invention is further exemplified by the following examples.

### Example 1

Detection of anti EP3 (PTGER3) in serum of patients with recurrent spontaneous abortions (RSA) using a Western blot

Jeg3 lysate was used as positive control as a reaction occurs if Jeg3 cells and serum of RSA patients is contacted.

EP3 protein was used as recombinant EP3 (PTGER3; Abnova; no. H00005733; lot 1010824). Recombinant EP3 has a molecular weight of 69,1 kD, whereas endogenous EP3 has a molecular weight of 43 kD.

As sample a pool of RSA positive sera was used.

For the Western blot, a Western blotting immunodetection kit of Vector Laboratories Inc. (30 Ingold Road, Burlingame, CA 94010) "Vector Stain^{®} ABC-AmP™" was used.

The solutions to be detected were the followings:
A): Jeg3 lysate (protein concentration = 1680 µg/mL) in two different reactions:
   A1: 8,3 µL Lysate + 21,7 µL aqua dest. + 10 µL Roti-Load buffer (Roti-Load 1, 4 x concentrate, Roth; no. K929.1; 10 mL)
   A2: 11,9 µL Lysate + 18,1 µL aqua dest. + 10 µL Roti-Load buffer
   B): recombinant EP3: 20 µL EP3 + 10 µL aqua dest. + 10 µL Roti-Load buffer

A gel was loaded as follows:
- lane1: Jeg3-lysate
- lane 2: Jeg3-lysate
- lane 3:: standard 5 µL
- lane 4:: EP3 - 18 µL
- lane 5:: 10 µg Jeg3-lysate - 18 µL
- lane 6:: 7 µg Jeg3-lysate - 18 µL

After loading the following steps were carried out:
i) 1 h incubation at room temperature with 1 x casein solution (10 x casein solution from kit 1:10 diluted in aqua dest.)
ii) incubation 16 h over night at 4°C with 3 mL pool of RSA positive sera (only lanes 5 to 7)
iii) wash step, 3 x 5 min in 1 x casein solution
iv) adding biotinylated goat-anti-human IgG + IgM (Jackson; no. 109-065-044; lot 94055), dilution 1:280 (= 5 µg/mL), and 45 min incubation at room temperature (only lanes 5 to 7) adding 20 µL + 5580 µL 1 x casein solution
v) wash step, 3 x 5 min in 1 x casein solution
vi) adding Vector Stain^{®} ABC-AmP™ reagent from the kit (10 µL A + 10 µL B per 5 mL 1 x casein solution) and incubating 20 min at room temperature
vii) washing step, 3 x 5 min in 1 x casein solution
viii) wash step, 5 min at room temperature with 0.1 M TRIS-buffer, pH 9.5
ix) adding BCIP/NBT-chromogen-substrate solution (10 mL 0.1 M TRIS-buffer pH 9.5 + 4 drops BCIP solution 1, mixing + 4 drops NBT solution 2 mixing + 4 drops MgCl₂ solution 3 mixing) and 3 min incubation at room temperature
x) stop reaction by addition of aqua dest. and
xi) dry membrane

After evaluation it can be seen that EP3 antibodies can be detected. Figure 1 shows the results for the identification of anti-EP3 antibodies (ATAB) by Western Blot.

In lane 4 EP3 antibodies are bound to recombinant EP3, in lanes 1, 2, 5 and 6 EP3 antibodies are bound to EP3 in the membrane of trophoblast cells Jeg3 for comparison.

### Example 2

### Dot blot

The following solutions were tested for EP3 antibodies:
Test solution 1: pool of RSA positive sera of IvF patients
Test solution 2: pool of RSA negative plasma of IvF patients
Test solution 3: pool of healthy controls

For detection reagents from Vector Stain^{®} ABC-AmP™ kit for Western blotting were used: Vector Stain^{®} ABC-Amp™ Western blotting immunodetection kit, rabbit-IgG kit (Vector, AK-6401). 10 µL EP3 (as in Example 1) with a concentration of 2 µg/mL were dotted on a nitrocellulose membrane. After drying the membrane the following steps were carried out:was blocked by 45 min incubation at room temperature with mL of 1 x casein solution.
i) block the membrane in 1 x casein solution for 45 minutes at room temperature
ii) remove solution
iii) add per dot 300 µL plasma (test solution 1, 2 or 3) (80 µL pooled plasma + 240 µL 1 x casein solution) and incubate at room temperature for 1 h; remove solution
iv) wash the membrane with 500 µL 1 x casein solution two times for 5 min each at room temperature
v) incubate the membrane for 1 h at room temperature in 300 µL per dot of goat-antihuman IgG + IgM (Dianova; no. 109-065-044; concentration: 1,4 mg/mL); dilution 1:280 (corresponding to 5 µg/mL) in 1 x casein solution = 22 µL + 6138 µL 1 x casein solution
vi) wash as in step iii)
vii) incubate the dots in ABS solution from ABC Amp kit (4 µL solution A + 4 µl solution B + 2 mL 1 x casein solution) for 20 min at room temperature
viii) wash as in step iii)
ix) incubate dots in substrate solution: BCIP/NBT from ABC Amp kit (5 mL 0.1 M TRIS + 2 drops solution 1 - mix - 2 drops solution 2 - mix - 2 drops solution 3) and incubate 10 min at room temperature
x) stop reaction with aqua dest. and dry the membrane.

The following results were obtained: Test solution 1 produced a signal, wheras no signal was found for test solutions 2 and 3.

### Example 3

### Dot blot

To evaluate if the autoantibodies found in samples from IVF patients were selective for EP3, another dot blot test was conducted wherein samples of RSA positive sera, samples of RSA negative sera and healthy controls were contacted with EP1, EP2, EP3 and EP4 respectively. The dot blot was carried out as described in Example 2. The proteins used were as follows:
EP1: PTGER1; Abnova; no. H00005731-P01; lot: 1010827; conc. 0.04 µg/µL; dilution: 1:20 = 3 µL + 57 µL PBS
EP2: PTGER2; Abnova; no. H00005732-P01; lot: 1010817; conc. 0.1 µg/mL; dilution: 1:50 = 2 µL + 98 µL PBS
EP3: PTGER3; Abnova; no. H00005733-P01; lot: 1010824; conc. 0.06 µg/µL; dilution: 1:30 = 2 µL + 58 µL PBS
EPq4: PTGER4; Abnova; no. H00005734-P01; lot: 1010824; conc. 0.03 µg/µL; dilution: 1:15 = 4 µL + 56 µL PBS

The results were as follows:

| Sample | Signal |
|---|---|
| Pool of RSA positive sera | EP3 positive |
| Sample of RSA positive patient | EP3 positve |
| Pool of RSA negative sera | EP3 negative |
| Sample of RSA negative patient | EP3 negative |
| Healthy control | EP3 negative |

## Claims

1. Method for the detection of EP3-autoantibodies, the method comprising contacting a sample with EP3 protein or an immunologically active fragment thereof and assessing for formation of an immune complex between the autoantibody and EP3.

2. A method of diagnosis of a risk for miscarriage and/or premature birth, wherein the method comprises:
a) contacting a sample with EP3 protein or an immunologically active fragment thereof and
b) detecting the presence of an immune complex between EP3 autoantibody
and EP3,
wherein the presence of EP3 autoantibodies is an indication for a risk for miscarriage and/or premature birth.

3. The method according to claim 1 or claim 2, wherein the immunologically active fragment is a peptide comprising at least one epitope of EP3.

4. The method of one of the preceding claims, wherein the method is a blotting method, such as dot blot or Western blot, or an immunometric method.

5. The method according to one of the preceding claims, wherein the method is a dot blot method and comprises
a) dotting EP3 protein or a fragment on a membrane,
b) adding an amount of a sample obtained from a patient to any dot,
c) adding antihuman IgG and/or antihuman IgM or a binding fragment thereof,
d) adding a detectable ligand,
e) determining the signals on the dot blot,
f) wherein a signal identifies a sample comprising EP3 autoantibody, wherein the method optionally further comprises washing and blocking steps.

6. Method according to one of claims 1 to 4, wherein the method is an immunometric assay and comprises
a) contacting a sample with immobilized EP3 to capture EP3 antibodies,
b) contacting the captured antibodies with conjugates comprising an antibody binding unit and a detectable unit,
c) detecting the presence and/or measuring the amount of detectable units
bound,
wherein the presence of detectable units identifies a sample comprising EP3 autoantibody.

7. Method according to one of the preceding claims, wherein the detection is carried out by adding antibodies reacting with conjugates comprising antihuman IgG and/or antihuman IgM coupled to an enzyme and a substrate for this enzyme.

8. Method according to one of the preceding claims, wherein the enzyme is alkaline phosphatase and the substrate is nitroblue tetrazolium chloride (NBT) or a fluorescent substrate or wherein the enzyme is horseradish peroxidase and the substrate is 3,3',5,5'-tetramethylbenzidine (TMB).

9. Kit for detection of EP3 autoantibodies comprising one or more EP3 proteins or immunologically active fragments thereof, conjugates comprising a ligand and a detectable unit and optionally buffer and/or wash solutions.

10. Kit for assessing a risk for miscarriage or premature birth comprising one or more EP3 proteins or fragments thereof, conjugates comprising an antibody binding unit and a detectable unit and optionally buffer and/or wash solutions.

11. Kit according to claim 8 or claim 9, wherein the antibody binding unit is antihuman IgG and/or antihuman IgM or a fragment thereof.

12. Kit according to one of claims 8 to 10, wherein the detectable unit is an enzyme and wherein the kit comprises a substrate for the enzyme.
